# EUROPÄISCHE PATENTSCHRIFT

(11) **EP 4 524 597 B1**
(45) Veröffentlichungstag und Bekanntmachung des Hinweises auf die Patenterteilung: **10.06.2026**
(21) Anmeldenummer: 23196927.0
(22) Anmeldetag: 12.09.2023
(51) Int. Cl.: G01R 33/565, G01R 33/561, G01R 33/36

(54) **VERFAHREN ZUR MAGNETRESONANZ-BILDGEBUNG, MAGNETRESONANZ-BILDGEBUNGSSYSTEM UND COMPUTERPROGRAMMPRODUKT**
METHOD FOR MAGNETIC RESONANCE IMAGING, MAGNETIC RESONANCE IMAGING SYSTEM AND COMPUTER PROGRAM PRODUCT
PROCÉDÉ D'IMAGERIE PAR RÉSONANCE MAGNÉTIQUE, SYSTÈME D'IMAGERIE PAR RÉSONANCE MAGNÉTIQUE ET PRODUIT DE PROGRAMME INFORMATIQUE

(43) Veröffentlichungstag der Anmeldung: 19.03.2025
(73) Patentinhaber: Siemens Healthineers AG, 91301 Forchheim (DE)
(72) Erfinder: Zeller, Mario, 91054 Erlangen (DE); Dispenza, Nadine, 91052 Erlangen (DE); Meixner, Christian, 91054 Erlangen (DE); Paul, Dominik, 91088 Bubenreuth (DE)
(74) Vertreter: Siemens Healthineers Patent Attorneys

(56) Entgegenhaltungen:
- CN-C- 100 346 171
- JONAS REBER ET AL.: "Correction of Gradient Induced Clock Phase Modulation for In-Bore Sampling Receivers", PROCEEDINGS OF THE INTERNATIONAL SOCIETY FOR MAGNETIC RESONANCE IN MEDICINE, ISMRM, 25TH ANNUAL MEETING AND EXHIBITION, HONOLULU, HI, USA, 22 APRIL - 27 APRIL 2017, no. 1056, 7 April 2017 (2017-04-07), XP040688624
- LE BIHAN D ET AL: "Artifacts and pitfalls in diffusion MRI", JOURNAL OF MAGNETIC RESONANCE IMAGING, SOCIETY FOR MAGNETIC RESONANCE IMAGING, OAK BROOK, IL, US, vol. 24, no. 3, 1 September 2006 (2006-09-01), pages 478 - 488, XP007913472, ISSN: 1053-1807, DOI: 10.1002/JMRI.20683

## Beschreibung

Magnetresonanz-, MR-, Bildgebungssysteme sind bildgebende Geräte, die ein starkes äußeres Magnetfeld nutzen, um die Kernspins eines zu untersuchenden Objekts auszurichten und sie durch Anlegen eines Anregungs-HF-Impulses zur Präzession um die entsprechende Ausrichtung anzuregen. Die Präzession beziehungsweise der Übergang der Spins von diesem angeregten Zustand in einen Zustand mit geringerer Energie erzeugt als Reaktion ein elektromagnetisches Wechselfeld, das über Empfangsantennen als MR-Signal detektiert werden kann.

Mit Hilfe von magnetischen Gradientenfeldern, beispielsweise als Gradientenpulse, kann den Signalen eine Positionskodierung aufgeprägt werden, die eine Zuordnung des empfangenen MR-Signals zu einem Volumenelement des zu untersuchenden Objekts ermöglicht. Die Gradientenpulse können in drei verschiedenen Raumrichtungen angelegt werden, die beispielsweise senkrecht aufeinander stehen und mit X-, Y-, und Z-Richtung bezeichnet werden. Einer üblichen Konvention zufolge ist die Z-Richtung parallel zum äußeren Grundmagnetfeld, sodass Gradientenpulse in Z-Richtung zur Auswahl einer abzubildenden Schicht im Objekt genutzt werden und auch als Schichtselektionsgradienten bezeichnet werden. In Y-Richtung findet in der genannten Konvention eine Phasenkodierung als Positionskodierung statt, und in X-Richtung eine Frequenzkodierung.

Das empfangene MR-Signal kann dann ausgewertet werden, um beispielsweise eine Bilddarstellung des Untersuchungsobjekts zu erhalten. Da das MR-Signal ein hochfrequentes Trägersignal aufweist, dessen Frequenz bei der Larmorfrequenz der Kernspins liegt, die eigentlich relevante Information jedoch im niederfrequenten Bereich liegt, beinhaltet das Auswerten der MR-Signale zur Bildrekonstruktion in der Regel eine Frequenzumsetzung zur Frequenzerniedrigung, beispielsweise durch Heruntermischen der MR-Signale mit der Larmorfrequenz als Mittenfrequenz.

Zum Empfangen der MR-Signale werden beispielsweise Auslesegradientenpulse geschaltet, wobei es sich insbesondere um Gradientenpulse in Frequenzkodierungsrichtung, also **X-**Richtung, handelt. Während der Auslesegradientenpulse wird in entsprechenden Auslesezeiträumen die Elektronik zum Auslesen des MR-Signals, die insbesondere einen Analog-zu-Digital Konverter, ADC (englisch: analog-to-digital converter), beinhaltet, aktiviert. Es wird zum Beispiel davon gesprochen, der ADC werde geschalten.

Die Auslesegradientenpulse sind beispielsweise näherungsweise rechteckige Pulse. Tatsächlich erstrecken sich die Flanken eines Auslesegradientenpulses jedoch in der Regel über einen, gegebenenfalls sehr kurzen Zeitraum. Eine typische Form eines Auslesegradientenpulses ist daher eine näherungsweise linear ansteigende Flanke, auch als Gradientenrampe oder Rampenphase bezeichnet, gefolgt von einem Bereich mit näherungsweise konstanter Amplitude, auch als Gradientenflachbereich (englisch: gradient flat top) oder Plateauphase bezeichnet, wiederum gefolgt von einer näherungsweise linear abfallenden Flanke.

Als sogenannte Glitches werden Störungen am Rand des k-Raums bezeichnet, von der gleichzeitigen Schaltung von ADC und numerisch gesteuerten Oszillatoren, auch als numerische Kristalloszillatoren bezeichnet, NCO (englisch: numerically controlled oscillator), herrühren können. Diese Störungen sind zeitlich an den Beginn des jeweiligen Auslesezeitzeitraums gekoppelt. Auch andere Störungen können zeitlich an den Beginn des jeweiligen Auslesezeitzeitraums gekoppelt sein.

Es ist eine Aufgabe der vorliegenden Erfindung bei der MR-Bildgebung Störeffekte zu reduzieren, insbesondere Störeffekte, die zeitlich an den Beginn eines jeweiligen Auslesezeitzeitraums gekoppelt sind.

Diese Aufgabe wird durch den jeweiligen Gegenstand der unabhängigen Ansprüche gelöst. Vorteilhafte Weiterbildungen und bevorzugte Ausführungsformen sind Gegenstand der abhängigen Ansprüche.

CN 100 346 171 C offenbart ein Magnetresonanzbildgebungsverfahren mit einer zufallsbedingten Verzögerung des Auslesezeitraums.

Die Erfindung beruht auf dem Gedanken, eine Zufallskomponente beim Auslesen beziehungsweise Verarbeiten der MR-Signale zu implementieren. Dazu werden verschiedene Auslesezeiträume zufällig oder pseudozufällig verzögert und/oder die Mittenfrequenz zur Frequenzumsetzung wird zufällig oder pseudozufällig verschoben.

Gemäß einem Aspekt der Erfindung wird ein Verfahren zur Magnetresonanz-, MR-Bildgebung angegeben. Dabei wird für jeden Auslesegradientenpuls einer Vielzahl von Auslesegradientenpulsen während eines dem jeweiligen Auslesegradientenpuls zugeordneten Auslesezeitraums mittels einer Empfangsantenne ein MR-Signal erfasst. Abhängig von den erfassten MR-Signalen, insbesondere allen der erfassten MR-Signale wird ein MR-Bild, insbesondere ein MR-Bild im Ortsraum, erzeugt, insbesondere rekonstruiert.

In einer ersten Variante wird ein Startzeitpunkt des jeweiligen Auslesezeitraums bezüglich eines Startzeitpunkts des jeweiligen zugeordneten Auslesegradientenpulses um eine Verzögerungsdauer verzögert, welche für jeden Auslesegradientenpuls der Vielzahl von Auslesegradientenpulsen zufällig oder pseudozufällig aus zwei oder mehr vorgegebenen Zeitwerten ausgewählt wird.

Gemäß einer zweiten Variante werden zum Erzeugen des MR-Bilds die erfassten MR-Signale jeweils verarbeitet, wobei die Verarbeitung eine Frequenzumsetzung, insbesondere eine Frequenzumsetzung zur Frequenzverringerung, gemäß einer Mittenfrequenz beinhaltet, wobei die Mittenfrequenz für jeden Auslesegradientenpuls der Vielzahl von Auslesegradientenpulsen zufällig oder pseudozufällig aus zwei oder mehr vorgegebenen Frequenzwerten ausgewählt wird.

Die erste Variante und die zweite Variante können auch kombiniert werden, sodass also die Startzeitpunkte der Auslesegradientenpulse um die zufällig oder pseudozufällig aus den zwei oder mehr vorgegebenen Zeitwerten ausgewählte Verzögerungsdauer verzögert wird und zusätzlich die Mittenfrequenz zur Frequenzumsetzung zufällig oder pseudozufällig aus zwei oder mehr vorgegebenen Frequenzwerten ausgewählt wird.

Insbesondere werden auch gemäß der ersten Variante zum Erzeugen des MR-Bilds die erfassten MR-Signale jeweils verarbeitet, wobei die Verarbeitung eine Frequenzumsetzung gemäß einer Mittenfrequenz beinhaltet. Die Mittenfrequenz kann in der ersten Variante jedoch für alle Auslesegradientenpulse dieselbe sein oder, wenn die erste mit der zweiten Variante kombiniert wird, wie beschrieben zufällig oder pseudozufällig aus den zwei oder mehr vorgegebenen Frequenzwerten ausgewählt werden.

Neben den genannten Auslesegradientenpulsen können auch weitere Gradientenpulse, beispielsweise zur Schichtauswahl, zur Positionscodierung und/oder zum Abtasten des k-Raums, sowie Hochfrequenzpulse zum Anregen, Refokussieren, Dephasieren, Umklappen und so weiter der Kernspins gemäß bekannter Messsequenzen geschaltet werden.

Ferner können mittels der Empfangsantenne in weiteren Auslesezeiträumen und/oder mittels weiterer Empfangsantennen weitere MR-Signale erfasst werden und das MR-Bild kann abhängig von den erfassten MR-Signalen und den erfassten weiteren MR-Signalen gemäß an sich, bis auf die zufällige oder pseudozufällige Auswahl der Zeitwerte und/oder der Frequenzwerte, bekannter Rekonstruktionsmethoden erzeugt werden.

Je nach Art der Messsequenz kann die Vielzahl von Auslesegradientenpulsen einer Vielzahl aufeinanderfolgender Auslesegradientenpulse innerhalb einer einzigen Messsequenz sein oder die Vielzahl von Auslesegradientenpulsen kann auf mehrere Messsequenzen verteilt sein. Es ist also auch möglich, dass eine Messsequenz jeweils nur einen der Auslesegradientenpulse beinhaltet.

Ein Auslesezeitraum beginnt mit dem entsprechenden Startzeitpunkt und endet zu einem entsprechenden Endzeitpunkt. Das MR-Signal wird für den entsprechenden Auslesezeitraum nur in der Zeit von dem Startzeitpunkt bis zu dem Endzeitpunkt erfasst beziehungsweise zur Rekonstruktion verwendet. Eine Gradientenamplitude eines Auslesegradientenpulses ist zu dem entsprechenden Startzeitpunkt des Auslesegradientenpulses insbesondere gleich null und zu einem entsprechenden Endzeitpunkt des jeweiligen Auslesegradientenpulses ebenfalls gleich null. Zwischen dem Startzeitpunkt und dem Endzeitpunkt des Auslesegradientenpulses kann die Gradientenamplitude von null verschieden sein. Es kann sich um einen unipolaren Puls handeln, sodass zwischen dem Startzeitpunkt und dem Endzeitpunkt kein Nulldurchgang der Gradientenamplitude vorliegt, oder um einen bipolaren Puls, bei dem ein solcher Nulldurchgang vorliegt. Der zeitliche Verlauf der Gradientenamplitude ist beispielsweise für alle Auslesegradientenpulse der Vielzahl von Auslesegradientenpulsen identisch. Bei bipolaren Auslesegradientenpulsen handelt es sich üblicherweise dennoch um zwei verschiedene Gradientenereignisse, wobei ein erster Teil und ein zweiter Teil des bipolaren Auslesegradientenpulses bis auf entgegengesetzt Amplituden gleich sind.

Insbesondere ist eine Dauer der Auslesegradientenpulse, also eine Zeitdifferenz zwischen dem entsprechenden Startzeitpunkt und dem Endzeitpunkt, für alle Auslesegradientenpulse der Vielzahl von Auslesegradientenpulsen gleich. Entsprechend ist auch eine Zeitdauer aller Auslesezeiträume, die den Auslesegradientenpulsen der Vielzahl von Auslesegradientenpulsen zugeordnet sind, also eine entsprechende Zeitdifferenz zwischen Startzeitpunkt und Endzeitpunkt des jeweiligen Auslesezeitraums, gleich. Beispielsweise ist auch die Anzahl von Messpunkten, auch als Abtastpunkte (englisch: sampling points) innerhalb eines Auslesezeitraums, also die entsprechende **Ab**tastrate, für alle Auslesezeiträume gleich.

Durch die Frequenzumsetzung wird das jeweilige MR-Signal insbesondere von einem Frequenzband, das um eine erste Zentralfrequenz zentriert ist, auf ein Frequenzband umgesetzt, das um eine zweite Zentralfrequenz zentriert ist, welche kleiner ist als die erste Zentralfrequenz. Die erste Zentralfrequenz entspricht insbesondere einer Larmorfrequenz und die zweite Zentralfrequenz entspricht der Differenz zwischen der ersten Zentralfrequenz und der Mittenfrequenz zur Frequenzumsetzung. Dadurch wird mittels der Frequenzumsetzung also insbesondere ein hochfrequenter Anteil des MR-Signals entfernt oder näherungsweise entfernt, sodass mit anderen Worten eine Einhüllende des MR-Signals bestimmt wird.

Die zwei oder mehr vorgegebenen Zeitwerte selbst sind nicht zufällig oder pseudozufällig, sondern fest vorgegeben. Lediglich die Auswahl der Verzögerungsdauer für die einzelnen Auslesezeiträume erfolgt zufällig oder pseudozufällig. Zum zufälligen oder pseudozufälligen Auswählen der Verzögerungsdauer aus den zwei oder mehr vorgegebenen Zeitwerten kann jedem Zeitwert ein Identifikator, beispielsweise eine Zahl von 1 bis **N,** wobei N der Gesamtanzahl der zwei oder mehr vorgegebenen Zeitwerte entspricht, zugeordnet werden. Mithilfe eines Zufallszahlengenerators oder eines Pseudozufallszahlengenerators wird dann eine zufällige beziehungsweise pseudozufällige Sequenz der Identifikatoren, beispielsweise Zahlen von 1 bis **N,** bestimmt und den Auslesezeiträumen entsprechend ihrer zeitlichen Abfolge zugewiesen. Dies gilt in entsprechenden Ausführungsformen analog für die Mittenfrequenzen.

Die Anzahl N der zwei oder mehr Zeitwerte ist nicht notwendigerweise gleich der Anzahl von Auslesezeiträumen beziehungsweise der Anzahl der Vielzahl von Auslesegradientenpulsen, sondern ist insbesondere kleiner. Dementsprechend kann verschiedenen Auslesezeiträumen dieselbe Verzögerungsdauer zugeordnet sein. Die Zeitwerte der zwei oder mehr vorgegebenen Zeitwerte sind insbesondere alle kleiner als eine vorgegebene Maximalverzögerung. Die Maximalverzögerung kann insbesondere derart gewählt werden, dass sich eine vorgegebene Reihenfolge der Auslesezeiträume bezüglich einander durch die unterschiedliche Verzögerungsdauer nicht verändert, insbesondere wenn mehrere der Auslesezeiträume innerhalb einer einzigen Messsequenz liegen. Die konkrete Auswahl der Maximalverzögerungsdauer hängt dann beispielsweise von einer gewünschten oder erforderlichen Zeitdifferenz zwischen den einzelnen Auslesezeiträumen ab. Insbesondere ist die maximale Verzögerungsdauer kleiner als die jeweilige Dauer der Auslesezeiträume, insbesondere kleiner oder gleich einem Zehntel, einem Zwanzigstel, einem Fünfzigstel oder einem Hundertstel der Dauer der Auslesezeiträume.

Die zwei oder mehr Zeitwerte sind voneinander jeweils verschieden. Einer der zwei oder mehr Zeitwerte kann auch gleich null sein. Die Verzögerungsdauer muss nicht notwendigerweise bezüglich des Startzeitpunkts des jeweiligen Auslesegradientenpulses definiert sein. Sie kann vielmehr auch bezüglich eines sonstigen charakteristischen Zeitpunkts des jeweiligen Auslesezeitraums definiert sein, beispielsweise bezüglich eines Umschaltzeitpunkts zwischen einer Gradientenrampe und einem Gradientenflachbereich, sofern jeweils vorgesehen. Nichtsdestotrotz ergibt sich stets eine Verzögerung der Startzeitpunkte des Auslesezeitraums und des zugehörigen Auslesegradientenpulses zueinander.

Sowohl die zufällige oder pseudozufällige Verzögerung der Auslesezeiträume als auch die zufällige oder pseudozufällige Verschiebung der Mittenfrequenz zur Frequenzumsetzung bewirken eine Inkohärenz der zur Rekonstruktion verwendeten Daten unterschiedlicher Auslesezeiträume zueinander, insbesondere zu einer Inkohärenz im k-Raum. Dies hat zur Folge, dass Störungen aufgrund von Phänomenen, die an den Beginn des jeweiligen Auslesezeitraums gekoppelt sind, die also immer gleichzeitig mit dem Startzeitpunkt des Auslesezeitraums oder immer zu einem bestimmten Zeitpunkt nach dem Startzeitpunkt des Auslesezeitraums beziehungsweise in einem begrenzten Zeitraum nach dem Startzeitpunkt des Auslesezeitraums, im k-Raum verschmiert werden. Dies wiederum führt dazu, dass die entsprechenden Artefakte und Störungen im rekonstruierten MR-Bild im Ortsraum weniger stark zutage treten.

Dabei sei darauf hingewiesen, dass die Verzögerung der Auslesezeiträume beziehungsweise die Verschiebung der Mittenfrequenz bei der Erzeugung des rekonstruierten MR-Bilds insbesondere berücksichtigt wird, sodass die die zur Rekonstruktion erforderlichen Daten, insbesondere in der Nähe des k-Raum-Zentrums, der einzelnen Teil-k-Räume in Ausleserichtung zueinander passen. Mit anderen Worten werden die jeweils aufgenommenen Daten insbesondere an die durch den Auslesegradientenpuls definierte Stelle im k-Raum gebracht und dann entsprechend zur Rekonstruktion verwendet. Da die Störungen zeitlich an den Beginn des jeweiligen Auslesezeitraums gekoppelt sind, befinden sich die resultierenden Artefakte daher für unterschiedliche Auslesezeiträume an unterschiedlichen Stellen im k-Raum.

Gemäß zumindest einer Ausführungsform der zweiten Variante oder einer Kombination der ersten und der zweiten Variante beinhaltet die Frequenzumsetzung ein jeweiliges Heruntermischen der erfassten MR-Signale gemäß der Mittenfrequenz.

Insbesondere wird das entsprechende MR-Signal einem Mischer zugeführt, dem auch ein Referenzsignal zugeführt wird, welches die Mittenfrequenz aufweist. Das Referenzsignal kann beispielsweise mittels eines Lokaloszillators erzeugt werden. Das Referenzsignal ist insbesondere ein Sinussignal oder näherungsweise ein Sinussignal mit der Mittenfrequenz. Ein solches Heruntermischen ist Bestandteil üblicher Verfahren zur Verarbeitung von MR-Signalen zur Rekonstruktion des MR-Bildes. Im Unterschied zu üblichen Verfahren wird erfindungsgemäß jedoch in den entsprechenden Ausführungsformen nicht dieselbe Mittenfrequenz, nämlich insbesondere die Larmorfrequenz, verwendet, sondern die Mittenfrequenz wird wie oben beschrieben zufällig oder pseudozufällig bestimmt. Dadurch kann die erfindungsgemäß angestrebte Inkohärenz mit Vorteil durch Anpassung vorhandener elektronischer Komponenten beziehungsweise Ergänzung dieser erreicht werden.

Gemäß zumindest einer Ausführungsform wird die jeweilige Mittenfrequenz als Summe einer vorgegebenen Larmorfrequenz und einer Frequenzdifferenz bestimmt, wobei die jeweilige Frequenzdifferenz zufällig oder pseudozufällig aus zwei oder mehr vorgegebenen Frequenzdifferenzwerten ausgewählt wird.

Durch die zufällige oder pseudozufällige Auswahl der Frequenzdifferenz wird somit auch die Mittenfrequenz zufällig oder pseudozufällig ausgewählt. Die resultierende Mittenfrequenz kann dabei je nach Wert der Frequenzdifferenz größer oder kleiner sein als die Larmorfrequenz. Die Frequenzdifferenzen können also positiv oder negativ sein. Auf diese Weise lässt sich in kontrollierter Art die erfindungsgemäß angestrebte Inkohärenz realisieren.

Die Absolutwerte der zwei oder mehr vorgegebenen Frequenzdifferenzwerte sind insbesondere jeweils kleiner als eine vorgegebene maximale Frequenzdifferenz. Die maximale Frequenzdifferenz ist kleiner als die Larmorfrequenz, beispielsweise kleiner oder gleich einem Zehntel, einem Zwanzigstel, einem Fünfzigstel oder einem Hundertstel der Larmorfrequenz. Dadurch wird der ursprünglich durch die Frequenzumsetzung angestrebte Effekt, nämlich die Extraktion der Einhüllenden des entsprechenden MR-Signals, nach wie vor in guter Näherung erreicht.

Gemäß zumindest einer Ausführungsform, insbesondere gemäß der ersten Variante oder einer Kombination der ersten mit der zweiten Variante, ist eine Anzahl der Auslesegradientenpulse der Vielzahl von Auslesegradientenpulsen größer als eine Anzahl der zwei oder mehr Zeitwerte.

Dadurch kann die erfindungsgemäß angestrebte Inkohärenz erzielt werden, insbesondere können die einzelnen Zeitwerte unterschiedlich genug voneinander definiert werden um die Inkohärenz zu erzielen, ohne für einzelne Auslesezeiträume eine möglicherweise zu große Zeitverschiebung einzuführen.

Die Anzahl der Auslesegradientenpulse kann beispielsweise im Bereich [5, 500] oder im Bereich [10, 500] oder im Bereich [20, 200] liegen. Die Anzahl der zwei oder mehr Zeitwerte kann dann beispielsweise im Bereich [3, 50] oder im Bereich [3, 20] liegen, wobei beispielsweise stets sichergestellt wird, dass die Anzahl der zwei oder mehr Zeitwerte kleiner ist als die Anzahl der Auslesegradientenpulse. Die Anzahl der zwei oder mehr Frequenzwerte kann ebenfalls beispielsweise im Bereich [3, 50] oder im Bereich [3, 20] liegen, wobei beispielsweise stets sichergestellt wird, dass die Anzahl der zwei oder mehr Frequenzwerte kleiner ist als die Anzahl der Auslesegradientenpulse.

Gemäß zumindest einer Ausführungsform folgen die Auslesegradientenpulse der Vielzahl von Auslesegradientenpulsen innerhalb einer einzigen Messsequenz aufeinander, insbesondere unmittelbar aufeinander.

Beispielsweise wird ein Hochfrequenzanregungspuls geschaltet und die Auslesegradientenpulse der Vielzahl von Auslesegradientenpulsen werden alle nach dem Hochfrequenzanregungspuls geschaltet und bevor ein weiterer Hochfrequenzanregungspuls geschaltet wird.

Bei solchen Messsequenzen mit einer Vielzahl von Auslesegradientenpulsen können zeitlich definierte oder zeitlich eingeschränkte Phänomene, die zu Störeffekten führen, beispielsweise verstärkt auftreten, sodass sich die Erfindung hier besonders vorteilhaft auswirkt.

Gemäß zumindest einer Ausführungsform weisen die Auslesegradientenpulse der Vielzahl von Auslesegradientenpulsen innerhalb der Messsequenz abwechselnde Polarität auf.

Bei den Auslesegradientenpulsen handelt es sich insbesondere also um unipolare Gradientenpulse mit abwechselnder Polarität innerhalb der Messsequenz.

Bei solchen Messsequenzen mit einer Vielzahl von Auslesegradientenpulsen können zeitlich definierte oder zeitlich eingeschränkte Phänomene, die zu Störeffekten führen, beispielsweise verstärkt auftreten, sodass sich die Erfindung hier besonders vorteilhaft auswirkt.

Gemäß zumindest einer Ausführungsform wird die Messsequenz als Echoplanarbildgebungsaufnahme, EPI-Aufnahme, (englisch: echoplanar imaging), oder als segmentierte EPI- Aufnahme durchgeführt.

Bei einer EPI-Aufnahme wird innerhalb einer einzigen Messsequenz der gesamte k-Raum einer gegebenen angeregten Schicht abgetastet. Bei einer segmentierten EPI-Aufnahme werden mehrere aufeinanderfolgende Messsequenzen verwendet, um den **k-**Raum einer angeregten Schicht abzutasten.

Bei solchen Messsequenzen mit einer Vielzahl von Auslesegradientenpulsen können zeitlich definierte oder zeitlich eingeschränkte Phänomene, die zu Störeffekten führen, beispielsweise verstärkt auftreten, sodass sich die Erfindung hier besonders vorteilhaft auswirkt.

Gemäß zumindest einer Ausführungsform weist jeder Auslesegradientenpuls der Vielzahl von Auslesegradientenpulsen eine erste Rampenphase auf, eine auf die erste Rampenphase folgende Plateauphase, und eine auf die Plateauphase folgende zweite Rampenphase. Der Startzeitpunkt des jeweiligen Auslesezeitraums liegt innerhalb der ersten Rampenphase und/oder der Endzeitpunkt des jeweiligen Auslesezeitraums liegt innerhalb der zweiten Rampenphase.

Die Plateauphase kann auch als Gradientenflachbereich bezeichnet werden und die Rampenphasen können als Gradientenrampen bezeichnet werden. Während der Plateauphase ist die Amplitude des Auslesegradientenpulses insbesondere konstant oder näherungsweise konstant gleich einem Plateauwert. Das Vorzeichen der Gradientenamplitude während der Plateauphase entspricht der Polarität des Auslesegradientenpulses beziehungsweise im Falle bipolarer Auslesegradientenpulse der Polarität des entsprechenden Teils des Auslesegradientenpulses. Während der ersten Rampenphase ändert sich die Gradientenamplitude insbesondere von null auf den Plateauwert, beispielsweise linear oder näherungsweise linear. Während der zweiten Rampenphase ändert sich die Gradientenamplitude von dem Plateauwert auf null, insbesondere linear oder näherungsweise linear.

Da der Startzeitpunkt des Auslesezeitraums innerhalb der ersten Rampenphase liegt und/oder der Endzeitpunkt des Auslesezeitpunkts innerhalb der zweiten Rampenphase liegt, wird also insbesondere eine Rampenabtastung (englisch: ramp sampling) genutzt. Dies ist insbesondere vorteilhaft, da dadurch mehr Zeit zur Signalerfassung zur Verfügung steht, als wenn die Signalerfassung nur während der Plateauphase erfolgen würde.

Gemäß zumindest einer Ausführungsform ändert sich die Amplitude des Auslesegradientenpulses während der ersten Rampenphase linear oder näherungsweise linear von null bis zu dem Plateauwert, ist während der Plateauphase konstant oder näherungsweise konstant gleich dem Plateauwert, und ändert sich während der zweiten Rampenphase linear oder näherungsweise linear von dem Plateauwert bis null.

Auf diese Weise lassen sich die Gradientenpulse besonders einfach erzeugen.

Näherungsweise linear und näherungsweise konstant können derart verstanden werden, dass ein linearer beziehungsweise konstanter Verlauf der Gradientenamplitude angestrebt oder eingestellt wird, der tatsächliche Verlauf jedoch aufgrund von Toleranzen oder sonstigen Variationen im üblichen Maß abweichen kann.

Gemäß einem weiteren Aspekt der Erfindung wird ein MR-Bildgebungssystem angegeben. Das MR-Bildgebungssystem weist eine Gradientenspulenanordnung auf, eine Empfangsantenne, eine mit der Empfangsantenne verbundene Signalerfassungseinheit sowie wenigstens eine Steuereinheit. Die wenigstens eine Steuereinheit ist dazu eingerichtet, die Gradientenspulenanordnung zum Erzeugen einer Vielzahl von Auslesegradientenpulsen anzusteuern und die Signalerfassungseinheit anzusteuern, für jeden Auslesegradientenpuls der Vielzahl von Auslesegradientenpulsen während eines jeweiligen Auslesezeitraums ein mittels der Empfangsantenne empfangenes MR-Signal zu erfassen. Das MR-Bildgebungssystem weist eine Auswerteeinheit auf, die dazu eingerichtet ist, abhängig von den erfassten MR-Signalen ein MR-Bild zu erzeugen, insbesondere zu rekonstruieren.

Gemäß einer ersten Variante ist die wenigstens eine Steuereinheit dazu eingerichtet, einen Startzeitpunkt des jeweiligen Auslesezeitraums bezüglich eines Startzeitpunkts des jeweiligen Auslesegradientenpulses um eine Verzögerungsdauer zu verzögern und die Verzögerungsdauer für jeden Auslesegradientenpuls zufällig oder pseudozufällig aus zwei oder mehr vorgegebenen Zeitwerten auszuwählen.

Gemäß einer zweiten Variante ist die wenigstens eine Steuereinheit dazu eingerichtet, die Signalerfassungseinheit anzusteuern, zur jeweiligen Verarbeitung der erfassten MR-Signale eine Frequenzumsetzung gemäß einer Mittenfrequenz durchzuführen und die Mittenfrequenz für jeden der Auslesegradientenpulse zufällig oder pseudozufällig aus zwei oder mehr vorgegebenen Frequenzwerten auszuwählen.

In verschiedenen Ausführungsformen können die erste und die zweite Variante des MR-Bildgebungssystems auch kombiniert sein.

Die wenigstens eine Steuereinheit beinhaltet insbesondere wenigstens eine Recheneinheit. Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "lookup table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß zumindest einer Ausführungsform weist die Signalerfassungseinheit einen Mischer auf, der dazu eingerichtet ist, zur Frequenzumsetzung die erfassten MR-Signale jeweils mit der Mittenfrequenz herunterzumischen.

Gemäß zumindest einer Ausführungsform weist das MR-Bildgebungssystem eine Hochfrequenzspulenanordnung auf, wobei die wenigstens eine Steuereinheit dazu eingerichtet ist, die Hochfrequenzspulenanordnung und die Gradientenspulenanordnung derart anzusteuern, dass die Auslesegradientenpulse der Vielzahl von Auslesegradientenpulsen in einer Messsequenz, insbesondere einer einzigen Messsequenz, aufeinanderfolgen, beispielsweise unmittelbar aufeinanderfolgen.

Gemäß zumindest einer Ausführungsform ist die wenigstens eine Steuereinheit dazu eingerichtet, die Hochfrequenzspulenanordnung und die Gradientenspulenanordnung derart anzusteuern, dass die Auslesegradientenpulse der Vielzahl von Auslesegradientenpulsen innerhalb der Messsequenz abwechselnde Polarität aufweisen.

Gemäß zumindest einer Ausführungsform ist die wenigstens eine Steuereinheit dazu eingerichtet, die Hochfrequenzspulenanordnung und die Gradientenspulenanordnung derart anzusteuern, dass die Messsequenz als EPI-Aufnahme, also als EPI-Messsequenz, oder als Messsequenz einer segmentierten EPI-Aufnahme durchgeführt wird.

Die wenigstens eine Steuereinheit kann in manchen Ausführungsformen auch die Signalerfassungseinheit beinhalten oder teilweise beinhalten und/oder die Auswerteeinheit beinhalten oder teilweise beinhalten.

Weitere Ausführungsformen des erfindungsgemäßen MR-Bildgebungssystems folgen unmittelbar aus den verschiedenen Ausgestaltungen des erfindungsgemäßen Verfahrens und umgekehrt. Insbesondere lassen sich einzelne Merkmale und entsprechende Erläuterungen sowie Vorteile bezüglich der verschiedenen Ausführungsformen zu dem erfindungsgemäßen Verfahren analog auf entsprechende Ausführungsformen des erfindungsgemäßen MR-Bildgebungssystems übertragen. Insbesondere ist das erfindungsgemäße MR-Bildgebungssystem zum Durchführen eines erfindungsgemäßen Verfahrens ausgebildet oder programmiert. Insbesondere führt das erfindungsgemäße MR-Bildgebungssystem das erfindungsgemäße Verfahren durch.

Gemäß einem weiteren Aspekt der Erfindung wird ein Computerprogramm mit Befehlen angegeben. Wenn die Befehle durch ein erfindungsgemäßes MR-Bildgebungssystem, insbesondere die wenigstens eine Steuereinheit des MR-Bildgebungssystems, ausgeführt werden, veranlassen die Befehle das MR-Bildgebungssystem dazu, ein erfindungsgemäßes Verfahren zur MR-Bildgebung durchzuführen.

Die Befehle können beispielsweise als Programmcode vorliegen. Der Programmcode kann beispielsweise als Binärcode oder Assembler und/oder als Quellcode einer Programmiersprache, zum Beispiel C, und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm speichert.

Das Computerprogramm und das computerlesbare Speichermedium sind jeweils Computerprogrammprodukte mit den Befehlen.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein, welche durch die Ansprüche definiert ist.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen:
- FIG 1: eine schematische Darstellung einer beispielhaften Ausführungsform des erfindungsgemäßen MR-Bildgebungssystems;
- FIG 2: eine schematische Darstellung einer MR-Aufnahme im k-Raum;
- FIG 3: eine schematische Darstellung eines MR-Bildes im Ortsraum;
- FIG 4: eine schematische Darstellung einer Messsequenz gemäß einer beispielhaften Ausführungsform des erfindungsgemäßen Verfahrens zur MR-Bildgebung;
- FIG 5: eine schematische Darstellung einer Messsequenz gemäß einer weiteren beispielhaften Ausführungsform des erfindungsgemäßen Verfahrens zur MR-Bildgebung;
- FIG 6: eine schematische Darstellung einer Messsequenz gemäß einer weiteren beispielhaften Ausführungsform des erfindungsgemäßen Verfahrens zur MR-Bildgebung;
- FIG 7: eine schematische Darstellung einer Messsequenz gemäß einer weiteren beispielhaften Ausführungsform des erfindungsgemäßen Verfahrens zur MR-Bildgebung; und
- FIG 8: eine schematische Darstellung einer Frequenzumsetzung gemäß einer weiteren beispielhaften Ausführungsform des erfindungsgemäßen Verfahrens zur MR-Bildgebung.

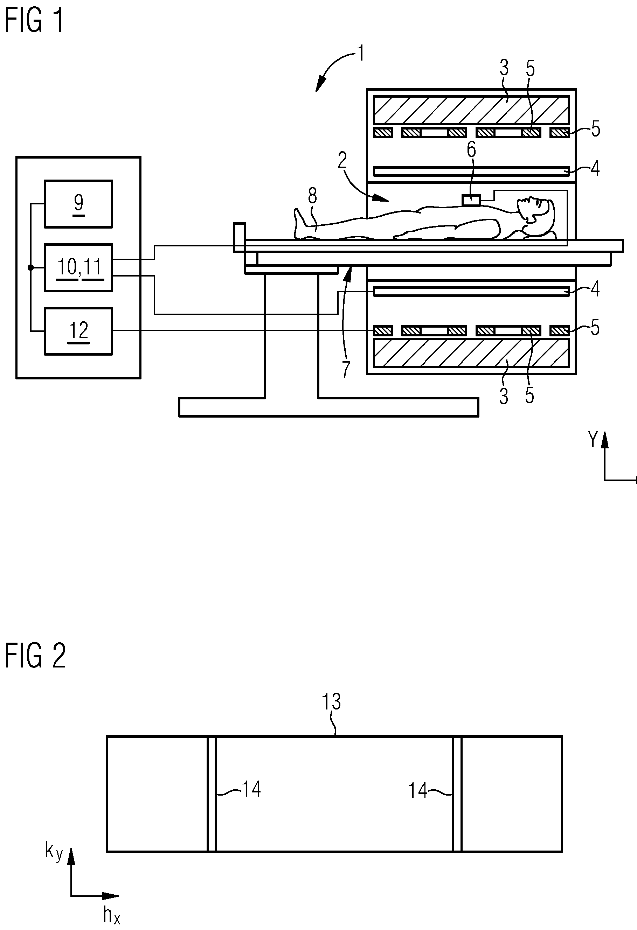

In FIG 1 ist eine beispielhafte Ausführungsform des erfindungsgemäßen MR-Bildgebungssystems 1 schematisch dargestellt.

Das MR-Bildgebungssystem 1 umfasst einen MR-Scanner mit einem Feldmagneten 3, der ein statisches Magnetfeld zur Ausrichtung der Kernspins eines abzubildenden Objekts 8 erzeugen kann. Der MR-Scanner kann einen Patiententunnel 2 aufweisen, die einen Abbildungsbereich umfasst, wobei das statische Magnetfeld in Bezug auf seine magnetische Feldstärke und/oder seinen Absolutwert innerhalb des Abbildungsbereichs äußerst homogen ist. Das Objekt 8 kann auf einem Patiententisch 7 angeordnet sein, der beweglich sein kann, um die Position des Objekts 8 zu verändern.

Der Feldmagnet 3 kann zum Beispiel ein supraleitender Magnet sein, also ein Elektromagnet mit einer supraleitenden Spule. Auf diese Weise können statische Magnetfelder bis zu 3T oder mehr erreicht werden. Für geringere Feldstärken können auch Permanentmagnete oder Elektromagnete mit normalleitenden Spulen verwendet werden.

Der MR-Scanner umfasst ferner eine Gradientenspulenanordnung 5, die jeweils variable Magnetfelder entlang jeder der drei räumlichen Dimensionen X, Y, Z erzeugen und die variablen Magnetfelder mit dem Grundmagnetfeld überlagern kann, um eine Positionskodierung zu erreichen. Bei den Gradientenspulen der Gradientenspulenanordnung 5 kann es sich um normalleitende Spulen. Insbesondere können die Gradientenspulen eine oder mehrere Gradientenspulen zur Erzeugung eines Magnetfeldgradienten in der X-Richtung, eine oder mehrere Gradientenspulen zur Erzeugung von Magnetfeldgradienten in der Y-Richtung und eine oder mehrere Gradientenspulen zur Erzeugung von Magnetfeldgradienten entlang der Z-Richtung umfassen.

Darüber hinaus kann der MR-Scanner eine oder mehrere Sendespulen 4 aufweisen, um Hochfrequenzpulse zur Anregung von Kernspins zur Präzessionsbewegung in dem entsprechenden Magnetfeld zu erzeugen. Die Sendespulen 4 können auch als Empfangsantennen fungieren, um MR-Signale als Reaktion auf die Kernspinanregung und Präzessionsresonanz zu empfangen. Alternativ oder zusätzlich können eine oder mehrere dedizierte Empfangsspulen 6, zum Beispiel Körperspulen, die in der unmittelbaren Umgebung des Objekts 8 angeordnet sind, verwendet werden.

Das MR-Bildgebungssystem 1 umfasst ferner ein Steuersystem, das eine oder mehrere Recheneinheiten und zusätzliche elektronische Komponenten zur Steuerung des MR-Scanners und/oder zur Verarbeitung der MR-Signale enthalten kann. Das Steuerungssystem weist eine Auswerteeinheit 9 auf, eine Signalerfassungseinheit 10, sowie wenigstens eine Steuereinheit 11, 12, zum Beispiel eine Hochfrequenzsteuerung 11 und eine Gradientensteuerung 12. Die Gradientensteuerung 12 ist beispielsweise dazu eingerichtet, die Gradientenspulen über Zuleitungen mit variablen Strömen zu versorgen, welche zeitlich koordiniert die gewünschten Gradientenfelder, insbesondere in Form von Gradientenpulsen, in dem Abbildungsbereich bereitstellen können. Die Hochfrequenzsteuerung 11 ist beispielsweise dazu eingerichtet, Hochfrequenzpulse, zum Beispiel Anregungspulse, mit vorgegebenen zeitlichen Verläufen, Amplituden und spektraler Leistungsverteilung zur Anregung einer Magnetresonanz der Kernspins zu erzeugen.

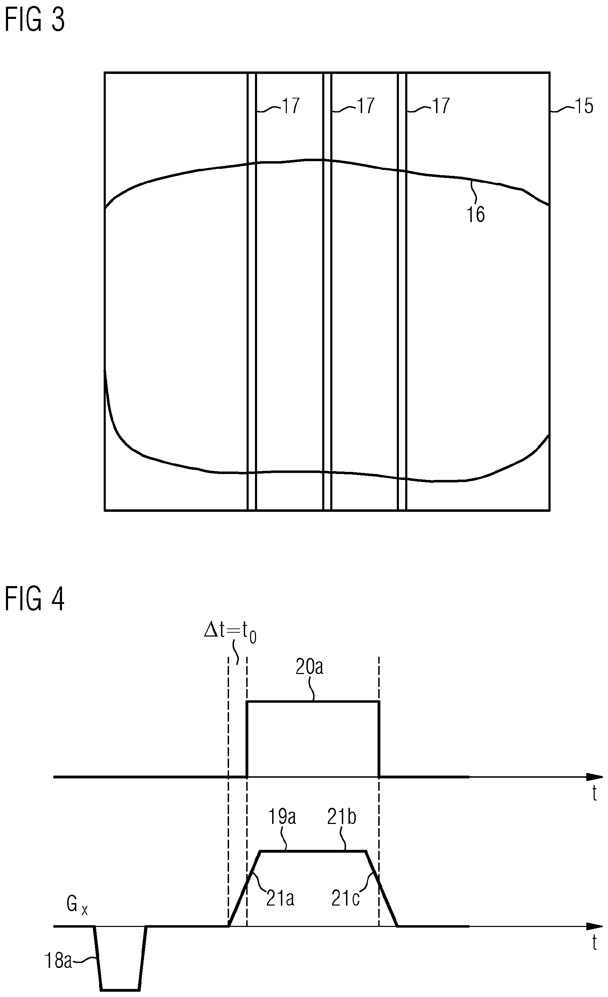

Mittels des MR-Bildgebungssystems 1 kann ein erfindungsgemäßes Verfahren durchgeführt werden. In den Figuren FIG 4 bis FIG 8 sind Einzelheiten verschiedener beispielhafter Ausführungsformen eines solchen erfindungsgemäßen Verfahrens schematisch dargestellt.

Die Gradientensteuerung 12 ist dazu eingerichtet, die Gradientenspulenanordnung 5 zum Erzeugen einer Vielzahl von Auslesegradientenpulsen 19a, 19b, 19c, 24a, 24b, 24c, 24d, 24e, 24f anzusteuern und die Signalerfassungseinheit 10 anzusteuern, für jeden Auslesegradientenpuls 19a, 19b, 19c, 24a, 24b, 24c, 24d, 24e, 24f während eines jeweiligen Auslesezeitraums 20a, 20b, 20c, 22a, 22b, 22c, 22d, 22e, 22f ein mittels einer der Empfangsantennen 4, 6 empfangenes MR-Signal zu erfassen. Die Auswerteeinheit 9 ist dazu eingerichtet ist, abhängig von den erfassten MR-Signalen ein MR-Bild zu erzeugen. Die Gradientensteuerung 12 ist dazu eingerichtet, um das MR-Bild zu erzeugen, zur jeweiligen Verarbeitung der erfassten MR-Signale eine Frequenzumsetzung gemäß einer Mittenfrequenz durchzuführen, um die MR-Signale herunterzumischen.

Die Gradientensteuerung 12 ist dazu eingerichtet, einen Startzeitpunkt des jeweiligen Auslesezeitraums 20a, 20b, 20c, 22a, 22b, 22c, 22d, 22e, 22f bezüglich eines Startzeitpunkts des jeweiligen Auslesegradientenpulses 19a, 19b, 19c, 24a, 24b, 24c, 24d, 24e, 24f um eine Verzögerungsdauer zu verzögern und die Verzögerungsdauer für jeden der Auslesegradientenpulse 19a, 19b, 19c, 24a, 24b, 24c, 24d, 24e, 24f zufällig oder pseudozufällig aus zwei oder mehr vorgegebenen Zeitwerten auszuwählen.

Alternativ oder zusätzlich ist die Signalerfassungseinheit 10 dazu eingerichtet, die Mittenfrequenz für jeden der Auslesegradientenpulse 19a, 19b, 19c, 24a, 24b, 24c, 24d, 24e, 24f zufällig oder pseudozufällig aus zwei oder mehr vorgegebenen Frequenzwerten auszuwählen.

Dadurch wird eine Inkohärenz der einzelnen Auswertezeiträume 20a, 20b, 20c, 22a, 22b, 22c, 22d, 22e, 22f bezüglich einander erzielt, welche Artefakte 14 im k-Raum verschmiert und damit entsprechende Artefakte 17 im Ortsraum wenigstens zum Teil kompensiert.

FIG 2 zeigt schematisch eine beispielhafte k-Raum Aufnahme 13 mit streifenförmigen Artefakten 14, die auftreten können, insbesondere wenn nicht ein erfindungsgemäßes Verfahren eingesetzt wird. FIG 3 zeigt schematisch ein zugehöriges MR-Bild 15 eines Objekts 16 im Ortsraum mit entsprechenden Artefakten 17.

In den Figuren FIG 4, FIG 5 und FIG 6 sind schematisch Sequenzdiagramme für drei Auslesegradientenpulse 19a, 19b, 19c und entsprechende Auslesezeiträume 20a, 20b, 20c gemäß einer beispielhaften Ausführungsform eines erfindungsgemäßen Verfahrens zur MR-Bildgebung dargestellt. Die Auslesegradientenpulse 19a, 19b, 19c entsprechen beispielsweise unterschiedlichen Messsequenzen. Während der zugehörigen Auslesezeiträume 20a, 20b, 20c wird jeweils ein MR-Signal empfangen und ein MR-Bild wird zumindest auch abhängig von den während der Auslesezeiträume 20a, 20b, 20c empfangenen MR-Signalen erzeugt. In der oberen Hälfte der FIG 4, FIG 5, FIG 6 ist als Funktion der Zeit t jeweils der Auslesezeitraum 20a, 20b, 20c dargestellt. In der unteren Hälfte ist jeweils die Gradientenamplitude G_{X} in Ausleserichtung beziehungsweise in Frequenzkodierungsrichtung dargestellt. Beispielsweise wird jeweils ein Dephasierungsgradientenpuls 18a, 18b, 18c, insbesondere mit negativer Polarität, geschalten und danach ein jeweiliger Auslesegradientenpuls 19a, 19b, 19c mit entgegengesetzter Polarität, insbesondere positiver Polarität. Während die Auslesegradientenpulse 19a, 19b, 19c geschalten werden, wird dementsprechend ein Gradientenechosignal erzeugt. Entsprechende Schichtselektionsgradienten in Z-Richtung beziehungsweise Gradienten zur Phasencodierung und so weiter in Y-Richtung sind der Übersichtlichkeit halber nicht dargestellt.

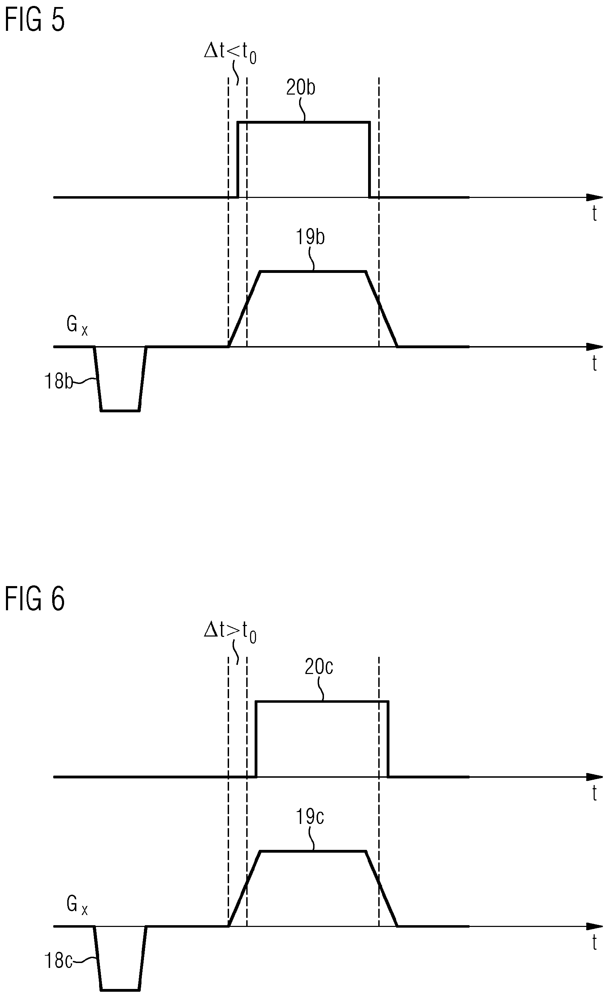

Die Auslesegradientenpulse 19a, 19b, 19c haben jeweils die gleiche Form und Dauer, also insbesondere denselben Amplitudenverlauf. Die Zeitdauern der Auslesezeiträume 20a, 20b, 20c sind ebenfalls gleich. Der jeweilige Startzeitpunkt des Auslesezeitraums 20a, 20b, 20c ist bezüglich eines Startzeitpunkts des zugehörigen Auslesegradientenpulses 19a, 19b, 19c um eine Verzögerungsdauer Δt verzögert. Die Verzögerungsdauern Δt sind dabei aus zwei oder mehr vorgegebenen Zeitwerten zufällig oder pseudozufällig ausgewählt. Im Beispiel der FIG 4 ist Δt gleich einer vorgegebenen nominellen Verzögerungszeit t0. Im Beispiel der FIG 5 ist Δt kleiner als t0 und im Beispiel der FIG 6 ist Δt größer als t0.

In den Beispielen der FIG 4, FIG 5, FIG 6 weisen die Auslesegradientenpulse 19a, 19b, 19c jeweils eine erste Rampenphase 21a, gefolgt von einer Plateauphase 21b, wiederum gefolgt von einer zweiten Rampenphase 21c auf. Beispielsweise befindet sich der Startzeitpunkt der Auslesezeiträume 20a, 20b, 20c jeweils in der ersten Rampenphase 21a und der Endzeitpunkt befindet sich in der zweiten Rampenphase 21c. Es wird also insbesondere eine Rampenabtastung durchgeführt.

Artefakte, die sich beispielsweise aufgrund von Störungen ergeben, die zeitlich an den Beginn des jeweiligen Auslesezeitraums gekoppelt sind, wie etwa Glitches, werden durch die zufällige beziehungsweise pseudozufällige Verzögerung der Auslesegradientenpulse 19a, 19b, 19c teilweise oder vollständig kompensiert.

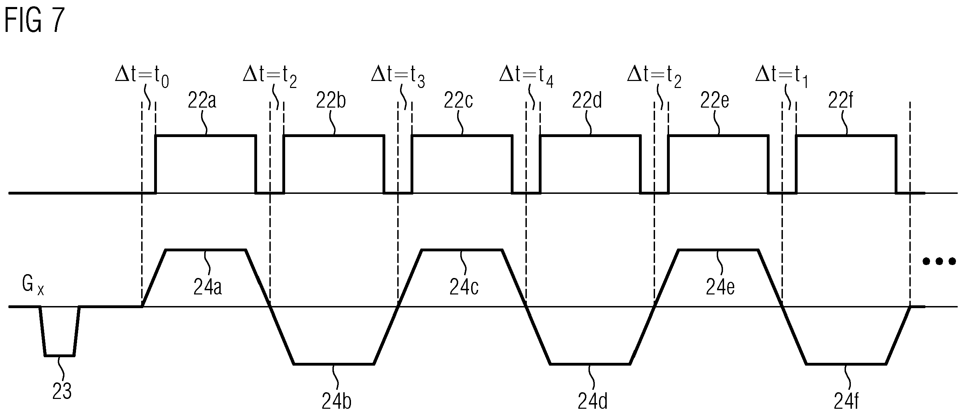

In der FIG 7 ist schematisch ein Sequenzdiagramm einer EPI-Messsequenz gemäß einer weiteren beispielhaften Ausführungsform eines erfindungsgemäßen Verfahrens dargestellt. Hier ist nach einem Dephasierungsgradientenpuls 23 eine Vielzahl direkt aufeinanderfolgender unipolarer Auslesegradientenpulse 24a, 24b, 24c, 24d, 24e, 24f mit abwechselnder Polarität vorgesehen. Die Form der einzelnen Auslesegradientenpulse 24a, 24b, 24c, 24d, 24e, 24f entspricht dabei beispielsweise der bezüglich FIG 4 bis FIG 6 beschriebenen Form. Auch hier ist jedem der Auslesegradientenpulse 24a, 24b, 24c, 24d, 24e, 24f ein Auslesezeitraum 22a, 22b, 22c, 22d, 22e, 22f zugeordnet, wobei ebenfalls beispielsweise Rampenabtastung durchgeführt wird. Für die Verzögerungsdauern Δt sind hier beispielsweise vier verschiedene Werte t0, t1, t2, t3 vorgesehen. Für die aufeinanderfolgenden Auslesegradientenpulse 24a, 24b, 24c, 24d, 24e, 24f beziehungsweise deren zugeordnete Auslesezeiträume 22a, 22b, 22c, 22d, 22e, 22f wird zufällig oder pseudozufällig jeweils einer der Werte t0, t1, t2, t3 ausgewählt. Im Beispiel der FIG 7 ist die Verzögerungsdauer Δt für den ersten dargestellten Auslesezeitraum 22a gleich t0, für den zweiten Auslesezeitraum 22b gleich t2, für den dritten Auslesezeitraum 22c gleich t3, für den vierten Auslesezeitraum 22d gleich t1, für den fünften Auslesezeitraum 22e gleich t2 und für den sechsten Auslesezeitraum 22f wiederum t1.

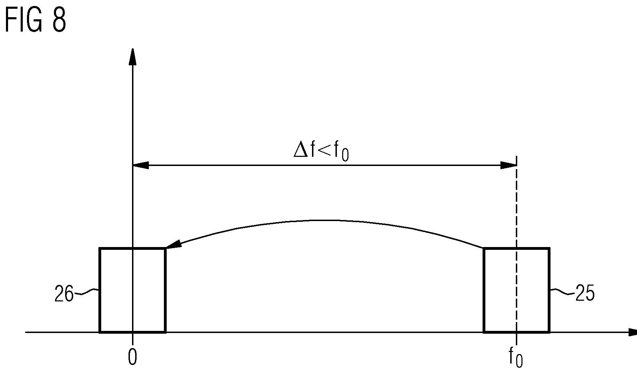

Alternativ oder zusätzlich zu der zufälligen Verzögerung der Startzeitpunkte der Auslesezeiträume 20a, 20b, 20c, 22a, 22b, 22c, 22d, 22e, 22f kann die Mittenfrequenz zur Frequenzumsetzung zum Heruntermischen der jeweiligen MR-Signale zufällig oder pseudozufällig ausgewählt werden, insbesondere um eine vorgegebene Larmorfrequenz herum. Dies ist schematisch in FIG 8 dargestellt. Das MR-Signal wird im Frequenzband 25 erfasst, welches beispielsweise um die Larmorfrequenz f₀ zentriert ist. Durch Heruntermischen mittels der Mittenfrequenz Δf, welche im gezeigten Beispiel exemplarisch kleiner ist als die Larmorfrequenz f₀, wird das Frequenzband 25 auf ein niedrigeres Frequenzband 26 umgesetzt, das um die Zentralfrequenz (f₀ - Δf) zentriert ist. Durch zufällige oder pseudozufällige Auswahl des Wertes für Δf wird die erfindungsgemäß angestrebte Inkohärenz ebenfalls erzielt beziehungsweise verstärkt.

Wie beschrieben, insbesondere bezüglich der Figuren, ermöglicht es die Erfindung, Störeffekte bei der MR-Bildgebung zu reduzieren.

In verschiedenen Ausführungsformen werden dazu die Startzeitpunkte der ADC-Aufnahmezeiträume in den einzelnen k-Raum Zeilen leicht gegeneinander verschoben, um eine inkohärente Verteilung möglicher Artefakte zu erreichen. Es wird insbesondere ein Verschmieren der Artefakt-Energie im k-Raum erreicht, was dementsprechend zu einer Reduzierung von Artefakten im rekonstruierten MR-Bild führt.

In verschiedenen Ausführungsformen wird eine Rampenabtastung durchgeführt. Bei der Rekonstruktion wird dann insbesondere ein sogenanntes Gridding-Verfahren angewendet. Dabei werden die auf den Gradientenrampen aufgenommenen Datenwerte wieder auf ein kartesisches Gitter interpoliert, da die folgenden Rekonstruktionsschritte, zum Beispiel eine Fouriertransformation, in der Regel kartesische Eingangsdaten erwarten. Im Rahmen des Gridding-Verfahrens werden die verschobenen Aufnahmen dann entsprechend ihrem tatsächlichen Startpunkt auf das kartesische Gitter interpoliert.

In verschiedenen Ausführungsformen kann die Anzahl der während eines Auslesezeitraums aufgenommenen Datenpunkte gegenüber einem nominalen Wert reduziert werden, beispielsweise um Beschränkungen der Datenaufnahme durch angrenzende Gradienten auf anderen Achsen Rechnung zu tragen. In anderen Ausführungsformen können die Auslesezeiträume auch gegenüber einer nominalen Dauer leicht verlängert werden, um weiterhin die vollständige Anzahl an Datenpunkten zur Verfügung zu haben.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Magnetresonanz-, MR-, Bildgebung, wobei für jeden Auslesegradientenpuls (19a, 19b, 19c, 24a, 24b, 24c, 24d, 24e, 24f) einer Vielzahl von Auslesegradientenpulsen (19a, 19b, 19c, 24a, 24b, 24c, 24d, 24e, 24f) während eines jeweiligen Auslesezeitraums (20a, 20b, 20c, 22a, 22b, 22c, 22d, 22e, 22f) mittels einer Empfangsantenne (4, 6) ein MR-Signal erfasst wird und abhängig von den erfassten MR-Signalen ein MR-Bild erzeugt wird, wobei
- ein Startzeitpunkt des jeweiligen Auslesezeitraums (20a, 20b, 20c, 22a, 22b, 22c, 22d, 22e, 22f) bezüglich eines Startzeitpunkts des jeweiligen Auslesegradientenpulses (19a, 19b, 19c, 24a, 24b, 24c, 24d, 24e, 24f) um eine Verzögerungsdauer verzögert wird, welche für jeden Auslesegradientenpuls (19a, 19b, 19c, 24a, 24b, 24c, 24d, 24e, 24f) zufällig oder pseudozufällig aus zwei oder mehr vorgegebenen Zeitwerten ausgewählt wird; und/oder
- zum Erzeugen des MR-Bilds die erfassten MR-Signale jeweils verarbeitet werden, wobei die Verarbeitung eine Frequenzumsetzung gemäß einer Mittenfrequenz beinhaltet, welche für jeden Auslesegradientenpuls (19a, 19b, 19c, 24a, 24b, 24c, 24d, 24e, 24f) zufällig oder pseudozufällig aus zwei oder mehr vorgegebenen Frequenzwerten ausgewählt wird.

2. Verfahren nach Anspruch 1, wobei die Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) in einer Messsequenz aufeinanderfolgen.

3. Verfahren nach Anspruch 2, wobei die Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) innerhalb der Messsequenz abwechselnde Polarität aufweisen.

4. Verfahren nach einem der Ansprüche 2 oder 3, wobei die Messsequenz als Echoplanarbildgebungsaufnahme oder als Messsequenz einer segmentierten Echoplanarbildgebungsaufnahme durchgeführt wird.

5. Verfahren nach einem der vorhergehenden Ansprüche, wobei
- jeder Auslesegradientenpuls (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) eine erste Rampenphase (21a), eine auf die erste Rampenphase (21a) folgende Plateauphase (21b), und eine auf die Plateauphase (21b) folgende zweite Rampenphase (21c) aufweist; und
- der Startzeitpunkt des jeweiligen Auslesezeitraums (20a, 20b, 20c, 22a, 22b, 22c, 22d, 22e, 22f) innerhalb der ersten Rampenphase (21a) liegt und/oder ein Endzeitpunkt des jeweiligen Auslesezeitraums (20a, 20b, 20c, 22a, 22b, 22c, 22d, 22e, 22f) innerhalb der zweiten Rampenphase (21c) liegt.

6. Verfahren nach Anspruch 5, wobei eine Amplitude des Auslesegradientenpulses (19a, 19b, 19c, 24a, 24b, 24c, 24d, 24e, 24f)
- sich während der ersten Rampenphase (21a) linear oder näherungsweise linear von Null bis zu einem Plateauwert ändert;
- während der Plateauphase (21b) konstant oder näherungsweise konstant gleich dem Plateauwert ist; und
- sich während der zweiten Rampenphase (21c) linear oder näherungsweise linear von dem Plateauwert bis Null ändert.

7. Verfahren nach einem der vorhergehenden Ansprüche, wobei
- eine Anzahl der Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) größer ist als eine Anzahl der zwei oder mehr Zeitwerte; und/oder
- die Anzahl der Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) größer ist als eine Anzahl der zwei oder mehr Frequenzwerte.

8. Verfahren nach einem der vorhergehenden Ansprüche, wobei die Frequenzumsetzung ein jeweiliges Heruntermischen der erfassten MR-Signale gemäß der Mittenfrequenz beinhaltet.

9. Verfahren nach einem der vorhergehenden Ansprüche, wobei die jeweilige Mittenfrequenz als Summe einer vorgegebenen Larmorfrequenz und einer Frequenzdifferenz bestimmt wird, wobei die jeweilige Frequenzdifferenz zufällig oder pseudozufällig aus zwei oder mehr vorgegebenen Frequenzdifferenzwerten ausgewählt wird.

10. MR-Bildgebungssystem (1) aufweisend eine Gradientenspulenanordnung (5), eine Empfangsantenne (4, 6), eine mit der Empfangsantenne (4, 6) verbundene Signalerfassungseinheit (10), wenigstens eine Steuereinheit (11, 12), die dazu eingerichtet ist, die Gradientenspulenanordnung (5) zum Erzeugen einer Vielzahl von Auslesegradientenpulsen (19a, 19b, 19c, 24a, 24b, 24c, 24d, 24e, 24f) anzusteuern und die Signalerfassungseinheit (10) anzusteuern, für jeden Auslesegradientenpuls (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) während eines jeweiligen Auslesezeitraums (20a, 20b, 20c, 22a, 22b, 22c, 22d, 22e, 22f) ein mittels der Empfangsantenne (4, 6) empfangenes MR-Signal zu erfassen, sowie eine Auswerteeinheit (9), die dazu eingerichtet ist, abhängig von den erfassten MR-Signalen ein MR-Bild zu erzeugen, wobei die wenigstens eine Steuereinheit (11, 12) dazu eingerichtet ist,
- einen Startzeitpunkt des jeweiligen Auslesezeitraums (20a, 20b, 20c, 22a, 22b, 22c, 22d, 22e, 22f) bezüglich eines Startzeitpunkts des jeweiligen Auslesegradientenpulses (19a, 19b, 19c, 24a, 24b, 24c, 24d, 24e, 24f) um eine Verzögerungsdauer zu verzögern und die Verzögerungsdauer für jeden der Auslesegradientenpulse (19a, 19b, 19c, 24a, 24b, 24c, 24d, 24e, 24f) zufällig oder pseudozufällig aus zwei oder mehr vorgegebenen Zeitwerten auszuwählen; und/oder
- die Signalerfassungseinheit (10) anzusteuern, zur jeweiligen Verarbeitung der erfassten MR-Signale eine Frequenzumsetzung gemäß einer Mittenfrequenz durchzuführen und die Mittenfrequenz für jeden der Auslesegradientenpulse (19a, 19b, 19c, 24a, 24b, 24c, 24d, 24e, 24f) zufällig oder pseudozufällig aus zwei oder mehr vorgegebenen Frequenzwerten auszuwählen.

11. MR-Bildgebungssystem (1) nach Anspruch 10, wobei die Signalerfassungseinheit (10) einen Mischer aufweist, der dazu eingerichtet ist, zur Frequenzumsetzung die erfassten MR-Signale jeweils gemäß der Mittenfrequenz herunterzumischen.

12. MR-Bildgebungssystem (1) nach einem der Ansprüche 10 oder 11, aufweisend eine Hochfrequenzspulenanordnung (4), wobei die wenigstens eine Steuereinheit (11, 12) dazu eingerichtet ist, die Hochfrequenzspulenanordnung (4) und die Gradientenspulenanordnung (5) derart anzusteuern, dass die Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) in einer Messsequenz aufeinanderfolgen.

13. MR-Bildgebungssystem (1) nach Anspruch 12, wobei die wenigstens eine Steuereinheit (11, 12) dazu eingerichtet ist, die Hochfrequenzspulenanordnung (4) und die Gradientenspulenanordnung (5) derart anzusteuern, dass die Auslesegradientenpulse (19a, 19b, 19c, 24a, 24b, 24c, 24d, 24e, 24f) der Vielzahl von Auslesegradientenpulsen (19a, 19b, 19c, 24a, 24b, 24c, 24d, 24e, 24f) innerhalb der Messsequenz abwechselnde Polarität aufweisen.

14. MR-Bildgebungssystem (1) nach einem der Ansprüche 12 oder 13, wobei die wenigstens eine Steuereinheit (11, 12) dazu eingerichtet ist, die Hochfrequenzspulenanordnung (4) und die Gradientenspulenanordnung (5) derart anzusteuern, dass die Messsequenz als Echoplanarbildgebungsaufnahme oder als Messsequenz einer segmentierten Echoplanarbildgebungsaufnahme durchgeführt wird.

15. Computerprogrammprodukt mit Befehlen, die bei Ausführung durch ein MR-Bildgebungssystem (1) nach einem der Ansprüche 10 bis 14 das MR-Bildgebungssystem (1) dazu veranlassen, ein Verfahren nach einem der Ansprüche 1 bis 9 durchzuführen.

## Claims

1. Method for magnetic resonance imaging, MRI, wherein for each readout gradient pulse (19a, 19b, 19c, 24a, 24b, 24c, 24d, 24e, 24f) of a plurality of readout gradient pulses (19a, 19b, 19c, 24a, 24b, 24c, 24d, 24e, 24f) during a respective readout period (20a, 20b, 20c, 22a, 22b, 22c, 22d, 22e, 22f), an MR signal is acquired by means of a receiving antenna (4, 6) and an MR image is generated as a function of the acquired MR signals, wherein
- a start time of the respective readout period (20a, 20b, 20c, 22a, 22b, 22c, 22d, 22e, 22f) is delayed relative to a start time of the respective readout gradient pulse (19a, 19b, 19c, 24a, 24b, 24c, 24d, 24e, 24f) by a delay duration which is randomly or pseudo-randomly selected from two or more predefined time values for each readout gradient pulse (19a, 19b, 19c, 24a, 24b, 24c, 24d, 24e, 24f); and/or
- to generate the MR image, the acquired MR signals are each processed, wherein the processing includes a frequency conversion based around a centre frequency selected randomly or pseudo-randomly from two or more predetermined frequency values for each readout gradient pulse (19a, 19b, 19c, 24a, 24b, 24c, 24d, 24e, 24f).

2. Method according to claim 1, wherein the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) are consecutive in a measurement sequence.

3. Method according to claim 2, wherein the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) have alternating polarity within the measurement sequence.

4. Method according to one of claims 2 or 3, wherein the measurement sequence is performed as an echo-planar imaging recording or as a measurement sequence of a segmented echoplanar imaging recording.

5. Method according to one of the preceding claims, wherein
- each readout gradient pulse (19a, 19b, 19c, 24a, 24b, 24c, 24d, 24e, 24f) of the plurality of readout gradient pulses (19a, 19b, 19c, 24a, 24b, 24c, 24d, 24e, 24f) has a first ramp phase (21a), a plateau phase (21b) following the first ramp phase (21a), and a second ramp phase (21c) following the plateau phase (21b); and
- the start time of the respective readout period (20a, 20b, 20c, 22a, 22b, 22c, 22d, 22e, 22f) is within the first ramp phase (21a) and/or an end time of the respective readout period (20a, 20b, 20c, 22a, 22b, 22c, 22d, 22e, 22f) is within the second ramp phase (21c).

6. Method according to claim 5, wherein an amplitude of the readout gradient pulse (19a, 19b, 19c, 24a, 24b, 24c, 24d, 24e, 24f)
- changes linearly or approximately linearly from zero to a plateau value during the first ramp phase (21a);
- is constantly or approximately constantly equal to the plateau value during the plateau phase (21b); and
- changes linearly or approximately linearly from the plateau value to zero during the second ramp phase (21c).

7. Method according to one of the preceding claims, wherein
- a number of the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) is greater than the number of the two or more time values; and/or
- the number of the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) is greater than the number of the two or more frequency values.

8. Method according to one of the preceding claims, wherein the frequency conversion comprises a respective downmixing of the acquired MR signals based around the centre frequency.

9. Method according to one of the preceding claims, wherein the respective centre frequency is determined as a sum of a predefined Larmor frequency and a frequency difference, wherein the respective frequency difference is selected randomly or pseudo-randomly from two or more predefined frequency difference values.

10. MR imaging system (1) having a gradient coil array (5), a receiving antenna (4, 6), a signal acquisition unit (10) connected to the receiving antenna (4, 6), at least one control unit (11, 12) which is designed to activate the gradient coil array (5) to generate a plurality of readout gradient pulses (19a, 19b, 19c, 24a, 24b, 24c, 24d, 24e, 24f) and to activate the signal acquisition unit (10) to acquire an MR signal received by means of the receiving antenna (4, 6) for each readout gradient pulse (19a, 19b, 19c, 24a, 24b, 24c, 24d, 24e, 24f) of the plurality of readout gradient pulses (19a, 19b, 19c, 24a, 24b, 24c, 24d, 24e, 24f) during a respective readout period (20a, 20b, 20c, 22a, 22b, 22c, 22d, 22e, 22f), and an evaluation unit (9) which is designed to generate an MR image based on the acquired MR signals, wherein the at least one control unit (11, 12) is designed to
- delay a start time of the respective readout period (20a, 20b, 20c, 22a, 22b, 22c, 22d, 22e, 22f) relative to a start time of the respective readout gradient pulse (19a, 19b, 19c, 24a, 24b, 24c, 24d, 24e, 24f) by a delay duration and to select the delay duration for each of the readout gradient pulses (19a, 19b, 19c, 24a, 24b, 24c, 24d, 24e, 24f) randomly or pseudo-randomly from two or more predefined time values; and/or
- to activate the signal acquisition unit (10) to perform frequency conversion based around a centre frequency for respective processing of the acquired MR signals, and to select the centre frequency for each of the readout gradient pulses (19a, 19b, 19c, 24a, 24b, 24c, 24d, 24e, 24f) randomly or pseudo-randomly from two or more predetermined frequency values.

11. MR imaging system (1) according to claim 10, wherein the signal acquisition unit (10) has a mixer which is designed to mix down the acquired MR signals based around the centre frequency for frequency conversion.

12. MR imaging system (1) according to one of claims 10 or 11, having a radiofrequency coil array (4), wherein the at least one control unit (11, 12) is designed to control the radiofrequency coil array (4) and the gradient coil array (5) such that the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) are consecutive in a measurement sequence.

13. MR imaging system (1) according to claim 12, wherein the at least one control unit (11, 12) is designed to control the radiofrequency coil array (4) and the gradient coil array (5) such that the readout gradient pulses (19a, 19b, 19c, 24a, 24b, 24c, 24d, 24e, 24f) of the plurality of readout gradient pulses (19a, 19b, 19c, 24a, 24b, 24c, 24d, 24e, 24f) have alternating polarity within the measurement sequence.

14. MR imaging system (1) according to one of claims 12 or 13, wherein the at least one control unit (11, 12) is designed to control the high frequency coil array (4) and the gradient coil array (5) such that the measurement sequence is performed as an echo-planar imaging recording or as a measurement sequence of a segmented echo-planar imaging recording.

15. Computer program product comprising commands which, when executed by an MR imaging system (1) according to one of claims 10 to 14, cause the MR imaging system (1) to carry out a method according to one of claims 1 to 9.

## Revendications

1. Procédé d'imagerie par résonance magnétique, RM, dans lequel pour chaque impulsion (19a, 19b, 19c, 24a, 24b, 24c, 24d, 24e, 24f) de gradient de lecture d'une pluralité d'impulsions (19a, 19b, 19c, 24a, 24b, 24c, 24d, 24e, 24f) de gradient de lecture, on détecte, au moyen d'une antenne (4, 6) de réception, un signal RM, pendant un laps de temps (20a, 20b, 20c, 22a, 22b, 22c, 22d, 22e, 22f) de lecture respectif, et on produit une image RM en fonction des signaux RM détectés, dans lequel
- on retarde d'une durée de retardement un instant de début du laps de temps (20a, 20b, 20c, 22a, 22b, 22c, 22d, 22e, 22f) respectif de lecture par rapport à un instant de début de l'impulsion (19a, 19b, 19c, 24a, 24b, 24c, 24d, 24e, 24f) respective de gradient de lecture, durée que l'on choisit pour chaque impulsion (19a, 19b, 19c, 24a, 24b, 24c, 24d, 24e, 24f) de gradient de lecture aléatoirement ou pseudo-aléatoirement dans deux ou plusieurs valeurs de temps données à l'avance ; et/ou
- pour la production de l'image RM, on traite respectivement les signaux RM détectés, dans lequel le traitement comprend un changement de fréquence conformément à une fréquence médiane que l'on choisit pour chaque impulsion (19a, 19b, 19c, 24a, 24b, 24c, 24d, 24e, 24f) de gradient de lecture aléatoirement ou pseudo-aléatoirement dans deux ou plusieurs valeurs de fréquence données à l'avance.

2. Procédé suivant la revendication 1, dans lequel des impulsions (19a, 19b, 19c, 24a, 24b, 24c, 24d, 24e, 24f) de gradient de lecture de la pluralité d'impulsions (19a, 19b, 19c, 24a, 24b, 24c, 24d, 24e, 24f) de gradient de lecture se succèdent en une séquence de mesure.

3. Procédé suivant la revendication 2, dans lequel les impulsions (19a, 19b, 19c, 24a, 24b, 24c, 24d, 24e, 24f) de gradient de lecture de la pluralité d'impulsions (19a, 19b, 19c, 24a, 24b, 24c, 24d, 24e, 24f) de gradient de lecture ont des polarités alternées dans la séquence de mesure.

4. Procédé suivant l'une des revendications 2 ou 3, dans lequel on effectue la séquence de mesure sous la forme d'un enregistrement d'imagerie plan à écho ou sous la forme d'une séquence de mesure d'un enregistrement d'imagerie plan à écho segmenté.

5. Procédé suivant l'une des revendications précédentes, dans lequel
- chaque impulsion (19a, 19b, 19c, 24a, 24b, 24c, 24d, 24e, 24f) de gradient de lecture de la pluralité d'impulsions (19a, 19b, 19c, 24a, 24b, 24c, 24d, 24e, 24f) de gradient de lecture a une première phase (21a) en rampe, une phase (21b) en plateau suivant la première phase (21a) en rampe et une deuxième phase (21c) en rampe suivant la phase (21b) en plateau ; et
- l'instant de début du laps de temps (20a, 20b, 20c, 22a, 22b, 22c, 22d, 22e, 22f) respectif de lecture se trouve dans la première phase (21a) en rampe et/ou l'instant de fin de l'espace de temps (20a, 20b, 20c, 22a, 22b, 22c, 22d, 22e, 22f) respectif de lecture se trouve dans la deuxième phase (21c) en rampe.

6. Procédé suivant la revendication 5, dans lequel une amplitude de l'impulsion (19a, 19b, 19c, 24a, 24b, 24c, 24d, 24e, 24f) de gradient de lecture
- se modifie pendant la première phase (21a) en rampe linéairement ou à peu près linéairement de zéro jusqu'à une valeur de plateau ;
- et pendant la phase (21b) en plateau est constante ou à peu près constante en étant égale à la valeur de plateau ; et
- se modifie pendant la deuxième phase (21c) en rampe linéairement ou à peu près linéairement de la valeur de plateau à zéro.

7. Procédé suivant l'une des revendications précédentes, dans lequel
- un nombre des impulsions (19a, 19b, 19c, 24a, 24b, 24c, 24d, 24e, 24f) de gradient de lecture de la pluralité des impulsions (19a, 19b, 19c, 24a, 24b, 24c, 24d, 24e, 24f) de gradient de lecture est plus grand qu'un nombre des deux ou plusieurs valeurs de temps ; et/ou
- le nombre des impulsions (19a, 19b, 19c, 24a, 24b, 24c, 24d, 24e, 24f) de gradient de lecture de la pluralité des impulsions (19a, 19b, 19c, 24a, 24b, 24c, 24d, 24e, 24f) de gradient de lecture est plus grand qu'un nombre des deux ou plusieurs valeurs de fréquence.

8. Procédé suivant l'une des revendications précédentes, dans lequel le changement de fréquence comprend un mélange respectif vers le bas, des signaux RM détectés, conformément à la fréquence médiane.

9. Procédé suivant l'une des revendications précédentes, dans lequel on détermine la fréquence respective médiane comme somme d'une fréquence de Larmor donnée à l'avance et d'une différence de fréquence, dans lequel on choisit la différence respective de fréquence aléatoirement ou pseudo-aléatoirement dans deux ou plusieurs valeurs de différence de fréquence données à l'avance.

10. Système (1) d'imagerie RM comportant un agencement (5) de bobines de gradient, une antenne (4, 6) de réception, une unité (10) de détection de signal connectée à l'antenne (4, 6) de réception, au moins une unité (11, 12) de commande, qui est agencée pour commander l'agencement (5) de bobines de gradient pour la production d'une pluralité d'impulsions (19a, 19b, 19c, 24a, 24b, 24c, 24d, 24e, 24f) de gradient de lecture et pour commander l'unité (10) de détection de signal, pour détecter, pour chaque impulsion (19a, 19b, 19c, 24a, 24b, 24c, 24d, 24e, 24f) de gradient de lecture de la pluralité d'impulsions (19a, 19b, 19c, 24a, 24b, 24c, 24d, 24e, 24f) de gradient de lecture, pendant un laps de temps (20a, 20b, 20c, 22a, 22b, 22c, 22d, 22e, 22f) respectif de lecture, un signal RM reçu au moyen de l'antenne (4, 6) de réception ainsi qu'une unité (9) d'évaluation, qui est agencée pour produire une image RM en fonction des signaux RM détectés, dans lequel la au moins une unité (11, 12) de commande est agencée pour
- retarder, d'une durée de retardement, un instant de début du laps de temps (20a, 20b, 20c, 22a, 22b, 22c, 22d, 22e, 22f) respectif de lecture par rapport à un instant de début de l'impulsion (19a, 19b, 19c, 24a, 24b, 24c, 24d, 24e, 24f) respective de gradient de lecture, et choisir la durée de retardement pour chaque impulsion (19a, 19b, 19c, 24a, 24b, 24c, 24d, 24e, 24f) de gradient de lecture aléatoirement ou pseudo-aléatoirement dans deux ou plusieurs valeurs de temps données à l'avance ; et/ou
- commander l'unité (10) de détection de signal pour le traitement respectif des signaux RM détectés, pour effectuer un changement de fréquence conformément à une fréquence médiane et choisir la fréquence médiane pour chaque impulsion (19a, 19b, 19c, 24a, 24b, 24c, 24d, 24e, 24f) de gradient de lecture aléatoirement ou pseudo-aléatoirement dans deux ou plusieurs valeurs de fréquence données à l'avance.

11. Système (1) d'imagerie RM suivant la revendication 10, dans lequel l'unité (10) de détection de signal a un mélangeur, qui est agencé pour abaisser par mélange les signaux RM détectés respectivement conformément à la fréquence médiane.

12. Système (1) d'imagerie RM suivant l'une des revendications 10 ou 11, comportant un agencement (4) de bobines de haute fréquence, dans lequel la au moins une unité (11, 12) de commande est agencée pour commander l'agencement (4) de bobines de haute fréquence et l'agencement (5) de bobines de gradient, de manière à ce que les impulsions (19a, 19b, 19c, 24a, 24b, 24c, 24d, 24e, 24f) de gradient de lecture de la pluralité des impulsions (19a, 19b, 19c, 24a, 24b, 24c, 24d, 24e, 24f) de gradient de lecture se succèdent en une séquence de mesure.

13. Système (1) d'imagerie RM suivant la revendication 12, dans lequel la au moins une unité (11, 12) de commande est agencée pour commander l'agencement (4) de bobines de haute fréquence et l'agencement (5) de bobines de gradient, de manière à ce que les impulsions (19a, 19b, 19c, 24a, 24b, 24c, 24d, 24e, 24f) de gradient de lecture de la pluralité d'impulsions (19a, 19b, 19c, 24a, 24b, 24c, 24d, 24e, 24f) de gradient de lecture aient une polarité alternée dans la séquence de mesure.

14. Système (1) d'imagerie RM suivant l'une des revendications 12 ou 13, dans lequel la au moins une unité (11, 12) de commande est agencée pour commander l'agencement (4) de bobines de haute fréquence et l'agencement (5) de bobines de gradient, de manière à ce que la séquence de mesure soit effectuée sous la forme d'un enregistrement d'imagerie plan à écho ou comme séquence de mesure d'un enregistrement d'imagerie plan à écho segmenté.

15. Produit de programme d'ordinateur, qui commande des instructions, qui, lors de leur exécution par un système (1) d'imagerie RM suivant l'une des revendications 10 à 14, font que le système (1) d'imagerie RM exécute un procédé suivant l'une des revendications 1 à 9.
